# EUROPEAN PATENT APPLICATION

(11) **EP 4 046 526 A1**
(43) Date of publication of application: **24.08.2022**
(21) Application number: 21158345.5
(22) Date of filing: 22.02.2021
(51) Int. Cl.: A44C 15/00, A44C 5/00, A45D 34/00, A61L 9/04, A61L 9/12

(54) **DEVICE AND JEWELRY**

(71) Applicant: Lahme, Ursula, 40699 Erkrath (DE)
(72) Inventor:
(74) Representative: Meyer zu Bexten, Elmar

(57) **Abstract**

Preferably capsular device (10) having
a container (11), such as a blister pack (11), containing a preferably gelatinous fragrance and
a preferably microporous membrane (12) covering the container (11) for evaporating the fragrance.

## Description

### Technical Field

The invention relates to the products as per the preamble of the independent claims.

### Background Art

PTL1, which falls under Article 54(3) of the European Patent Convention and is hereby incorporated by reference, discloses a bracelet wherein a felt for absorbing a fragrance such as perfume or essential oil is retained. A decorative receptacle, such as of precious metal or stainless steel galvanized with rose or yellow gold, is provided for removably receiving the retainer.

Controlled release of fragrance from blister packs through microporous membranes - optionally sealed, for instance, by aluminum foil - is well understood (cf. NPL1).

### Summary of invention

The invention is defined by Claim 1.

### Advantageous effect of invention

The device can be used with compatible jewelry and easily replaced when the fragrance is depleted.

### Brief description of drawings

Figure 1 and Figure 2 are exploded views of a first bracelet.
Figure 3 and Figure 4 are views of the first bracelet's blister pack.
Figure 5, Figure 6, and Figure 7 are exploded views of a second bracelet.
Figure 8 is a view of the second bracelet's retainer.
Figure 9 and Figure 10 are views of the second bracelet's receptacle.
Figure 11, Figure 12, and Figure 13 are views of the second bracelet's capsular device.
Figure 14 is a view of the device's retainer in a nonfragrant pivotal position.
Figure 15 is a view of the device's retainer in a fragrant pivotal position.
Figure 16 is an exploded view of a third bracelet.
Figure 17 and Figure 18 are exploded views of a fourth bracelet.
Figure 19 and Figure 20 are views of the fourth bracelet's capsular device.
Figure 21, Figure 22, Figure 23, and Figure 24 are views of a fifth bracelet's capsular device.

### Description of embodiments

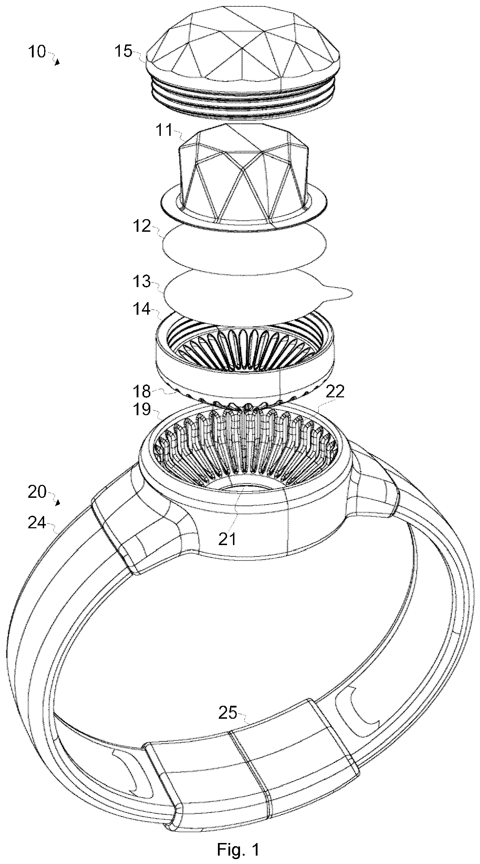

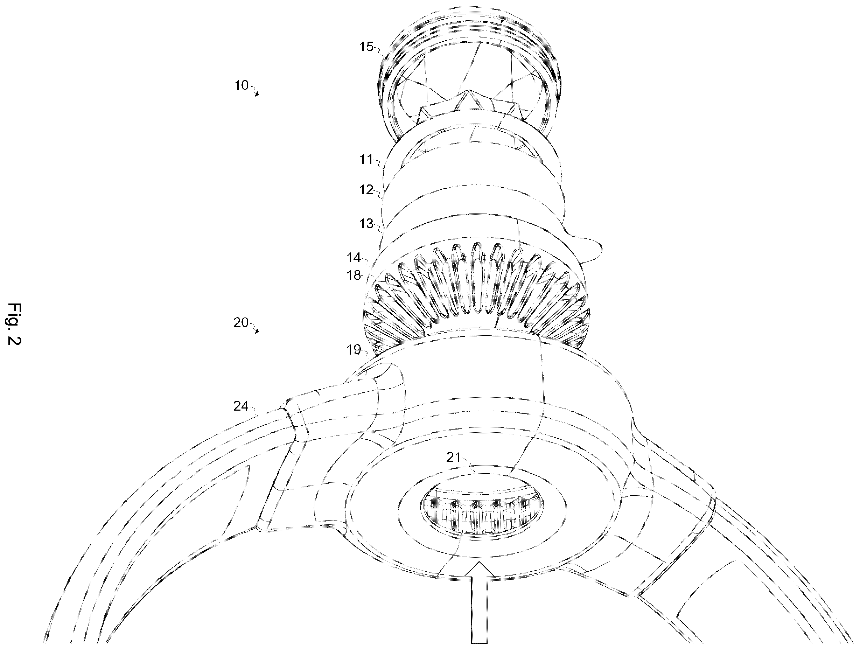

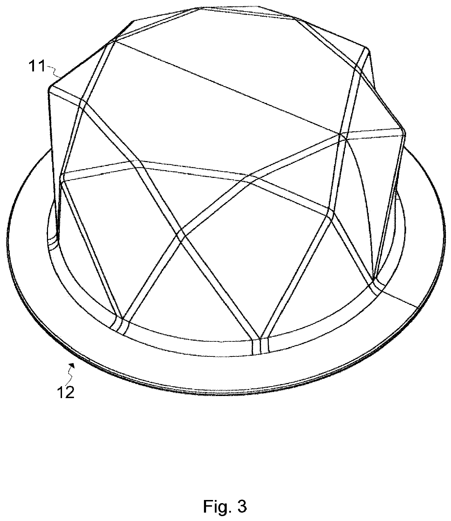

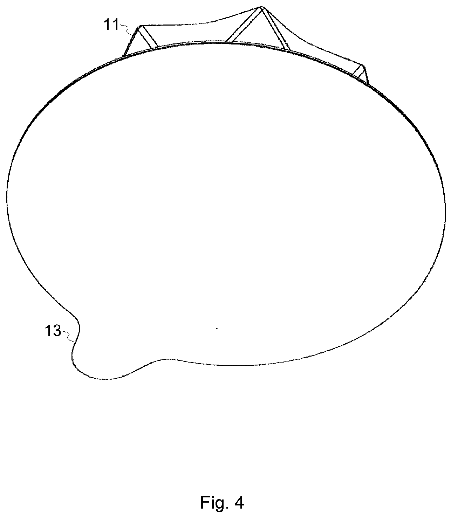

Figure 1 and Figure 2 depict a bracelet (20) having a metallic receptacle (19) that receives a capsular device (10). The device (10) contains a blister pack (11) filled with a preferably gelatinous fragrance based on perfume or essential oil and covered by a microporous membrane (12 - Figure 3). To prevent the fragrance from escaping the blister pack (11) through the thin membrane (12) prior to use, a peelable seal (13 - Figure 4) initially covers the membrane (12).

On its underside, the blister pack (11) is inserted in a rigid plastic retainer (14) bulging outward away from the membrane (12) and seal (13). The retainer (14) exhibits slight gaps (18) of arbitrary number, size, and layout which, as soon as the seal (13) is removed, release the fragrance slowly evaporating through the membrane (12). A transparent or opaque capsule (15) of glass or polycarbonate is screwed into the retainer (14) and encapsulates the blister pack (11) secured therein, thus completing the device (10).

Depicted below the device (10), a resizable wristband (24) with a magnetic or other clasp (25) attaches bilaterally to a metallic receptacle (19) bulging inward complementarily to the retainer (14) and receiving the latter, for instance, in a snap-in connection. When in use, a multitude of air ducts (22) formed in the receptacle (19) discharge the fragrance released through the gaps (18) of the retainer (14), which process is promoted by the wearer's body heat. As is best seen in Figure 2, a central opening (21) is formed in the receptacle (19), allowing to snap the device (10) out of the receptacle (19) to replace or refill it.

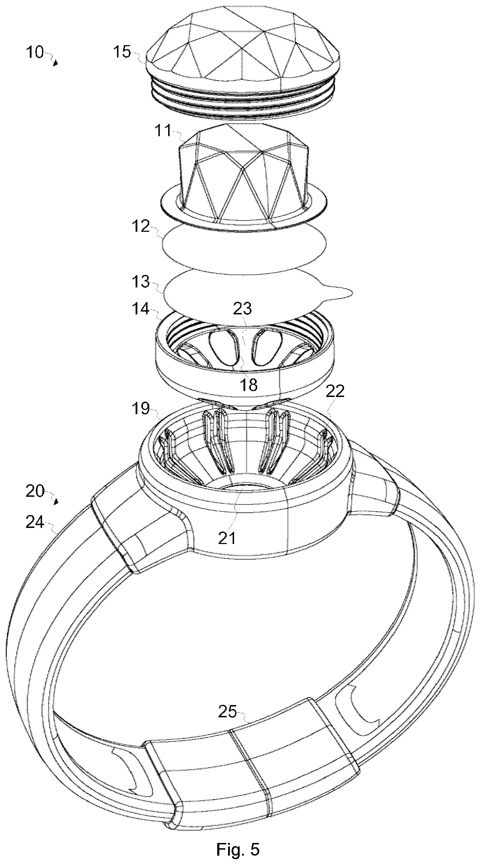

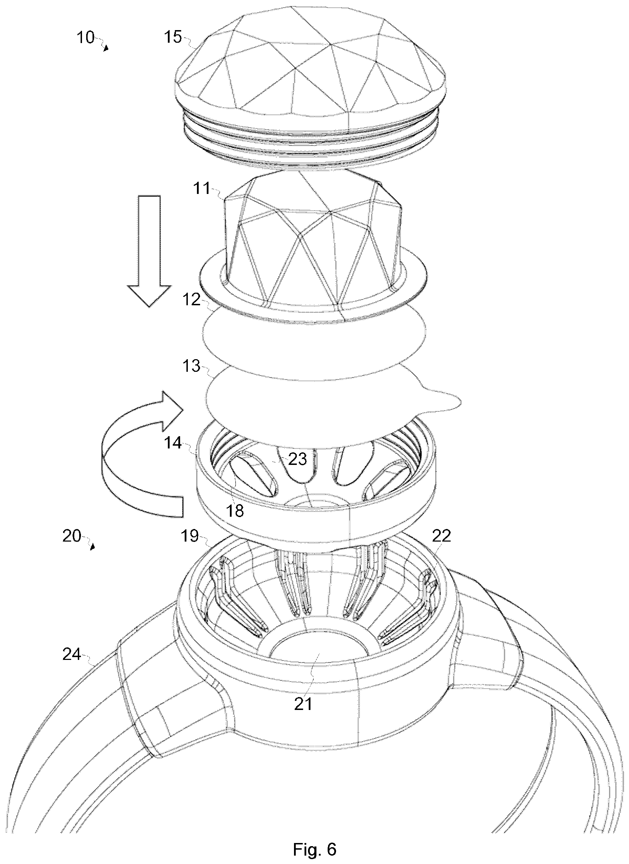

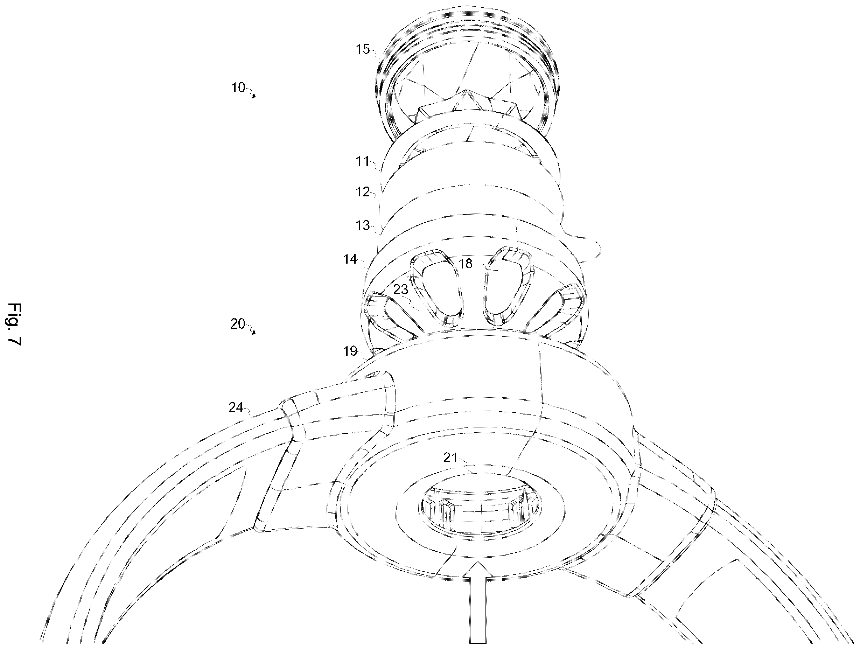

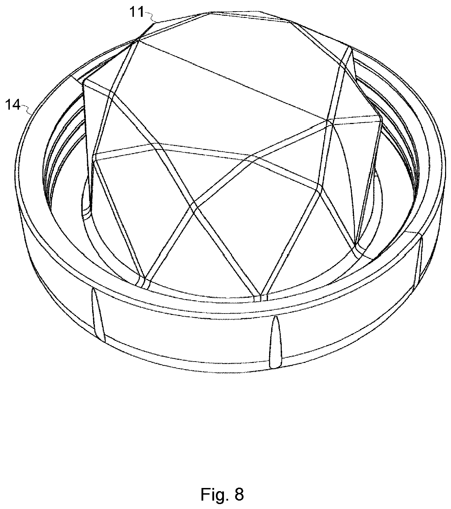

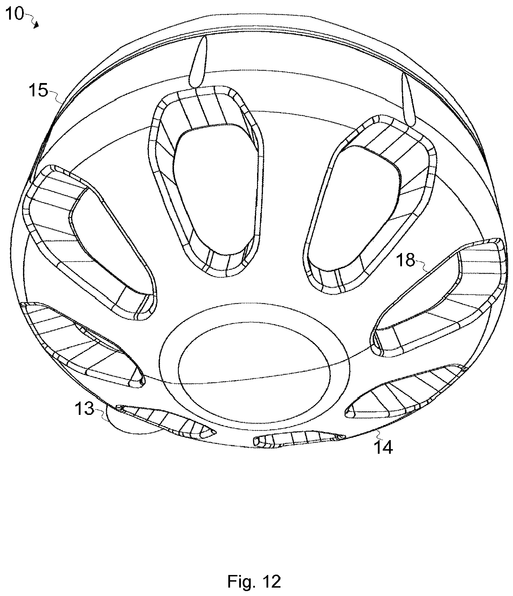

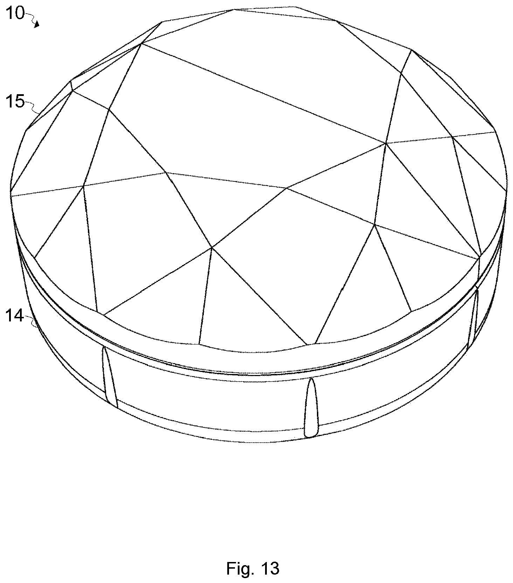

Figure 5 through Figure 15 depict a second bracelet (20) and its constituents. In this variant, release of the fragrance may be mechanically controlled by the bracelet's wearer. To this end, as illustrated in Figure 6, the receptacle (19) supports the capsular device (10) pivotably about the longitudinal axis shared by the receptacle (19) and retainer (14). In the present example, the latter exhibits only eight gaps (18), with each two adjacent gaps (18) being separated by a substantial stay (23).

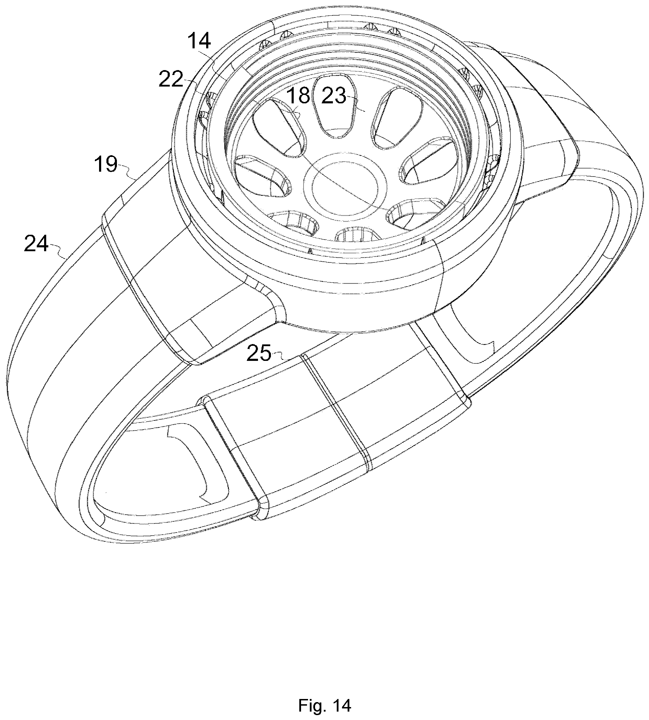

In the pivotal position of the device (10) shown in Figure 14, the arrangement of gaps (18) and stays (23) causes the gaps (18) to misalign with the ducts (22) of the receptacle (19). Due to the form fit present between the receptacle (19) and retainer (14), the latter's stays (23) thus block the ducts (22), withholding the otherwise evaporating fragrance.

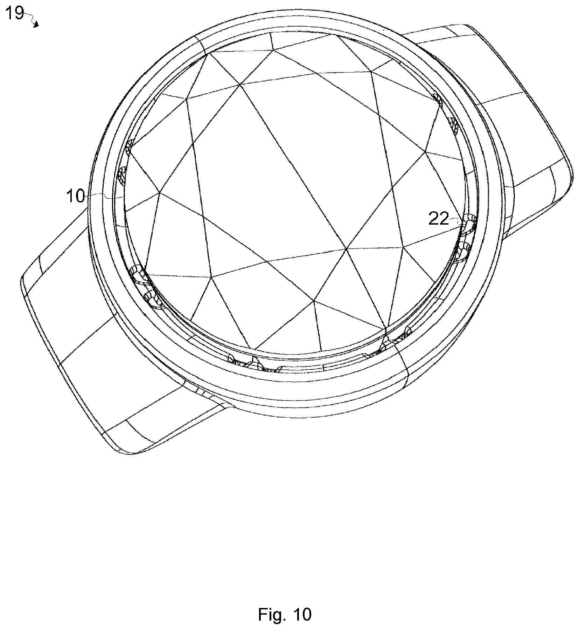

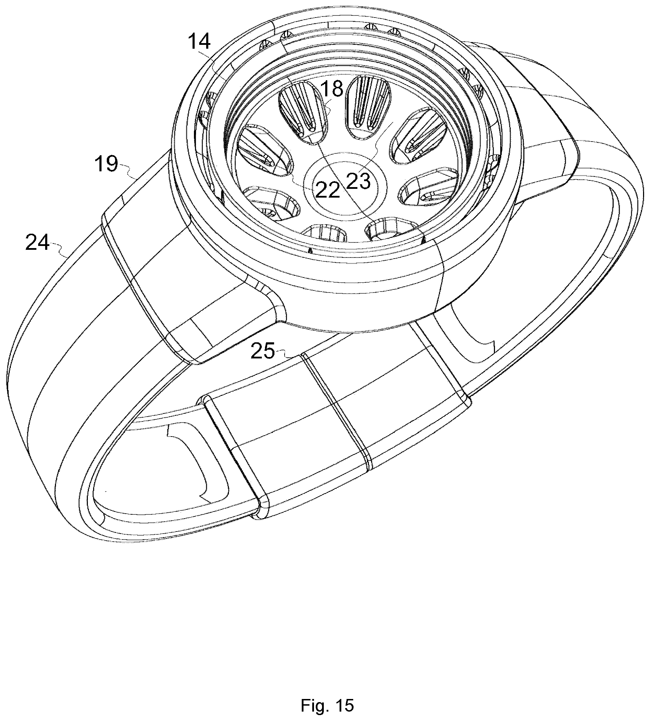

When the user pivots the device (10) into the alternative position shown in Figure 15, the gaps (18) align with the ducts (22) to release the fragrance. Correspondingly to the first bracelet (20), and best seen in Figure 10, the ducts (22) extend alongside the device (10) and open out peripherally opposite its membrane (12) where they discharge the fragrance into the ambient air. Upon depletion of the blister's contents, the capsular device (10) may be replenished or substituted for a newly purchased unit.

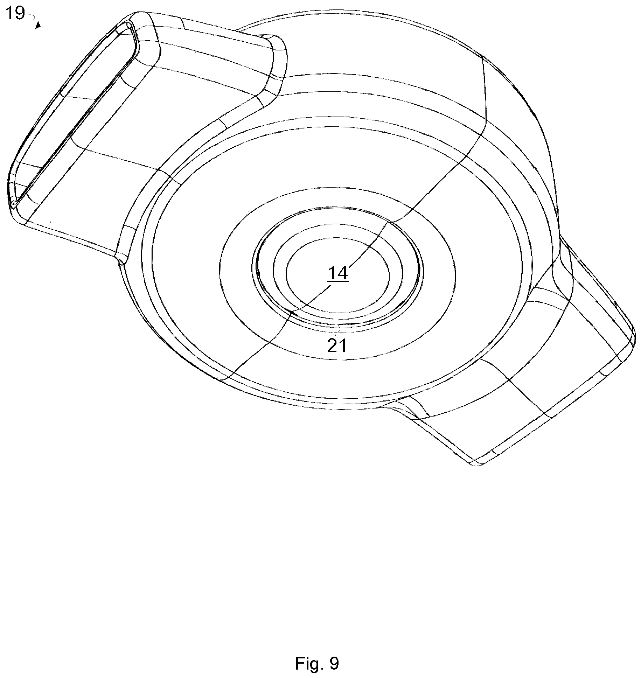

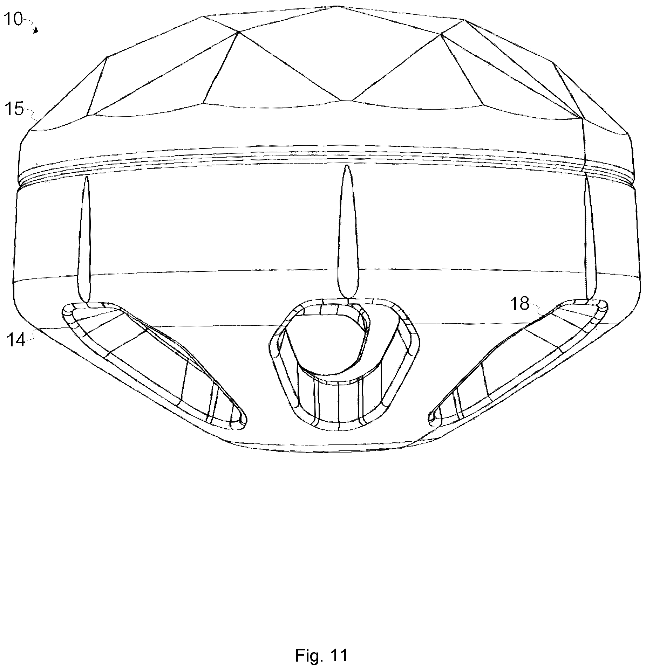

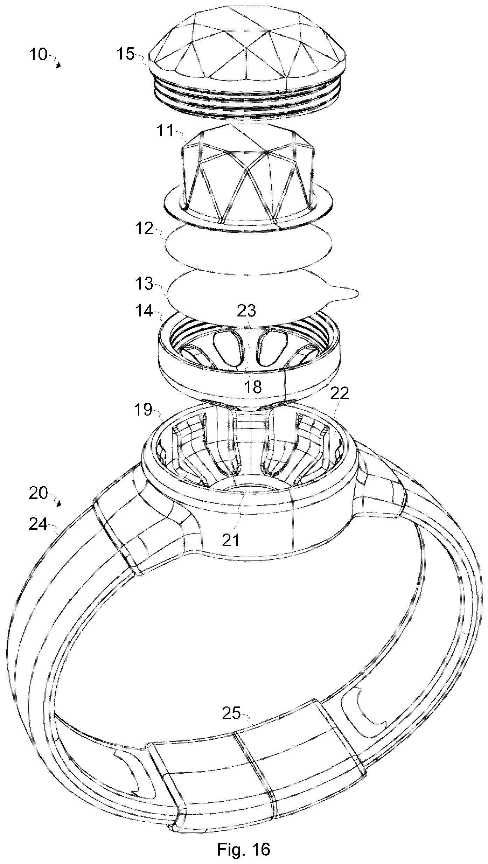

The skilled person will appreciate that a receptacle (19) akin to that of Figure 9 and Figure 10 could be integrated in a necklace or other jewelry, permitting the device (10) of Figure 11 through Figure 13 to be employed in a modular fashion. It is also apparent that the air ducts (22) of the receptacle (19), much like the gaps (18) formed in the retainer (14), may vary in number and shape while still providing the desired control over the release of fragrance. By way of example, a third bracelet (20) whose number of ducts (22) coincides with the number of gaps (18) and stays (23) of the retainer (14) is shown in Figure 16.

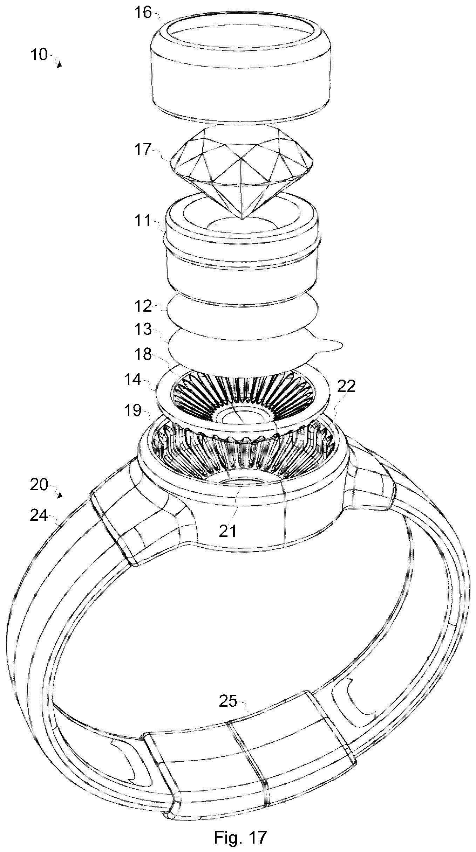

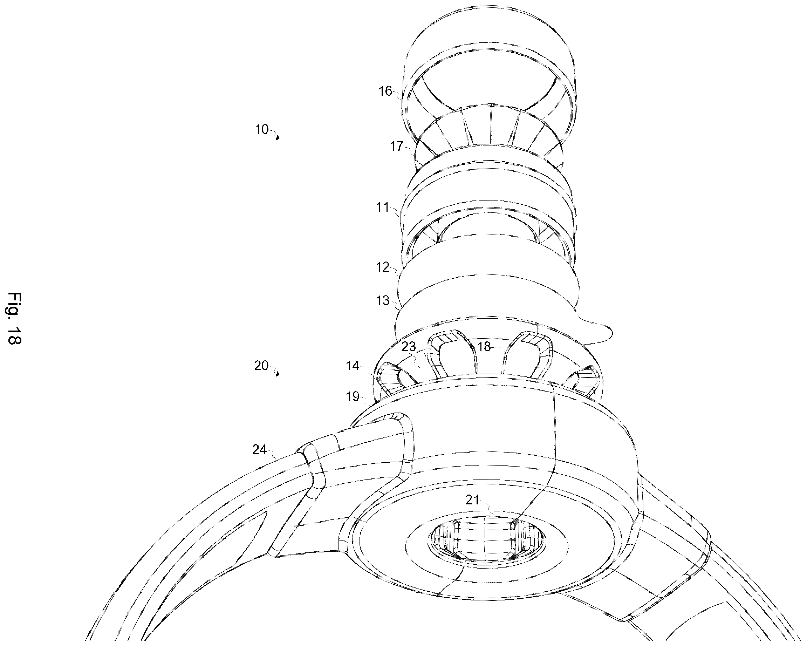

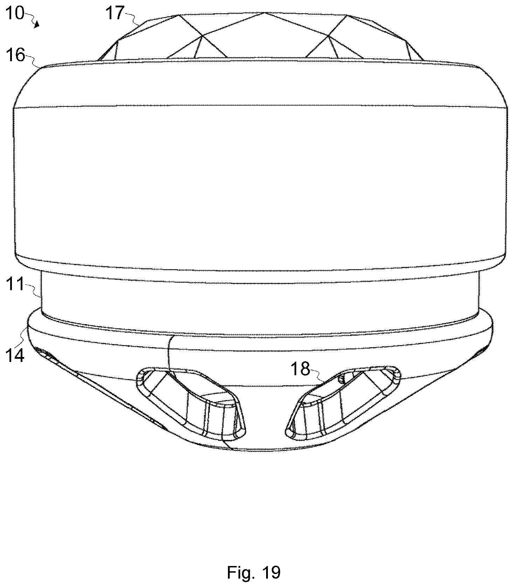

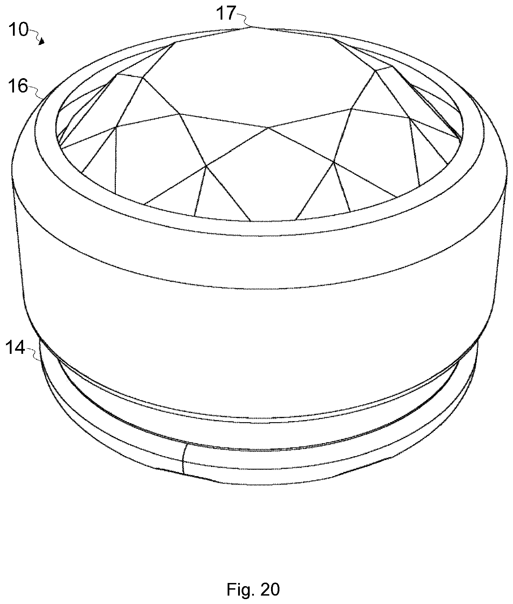

Similarly, encapsulation of the device (10) may be effected by other means, one of which is elucidated in Figure 17 through Figure 20. The device (10) of this fourth bracelet (20) is adorned, instead of the low-cost capsule (15) of the previous devices, by a gemstone or rhinestone (17) encompassed in a bezel (16) joined to the retainer (14) opposite the membrane (12), for example, by screwing or clipping via the cylindrical fragrance container (11).

Yet another option (not depicted) makes use of an elastic ring, such as of rubber, snapped onto or into the retainer (14) opposite the membrane (12). By means of a circumferential groove, the rhinestone (17) of this device (10) in turn snaps into the ring, resulting in a detachable connection between stone (17) and retainer (14). In PTL1, a connection of this type and its advantage are discussed in further detail.

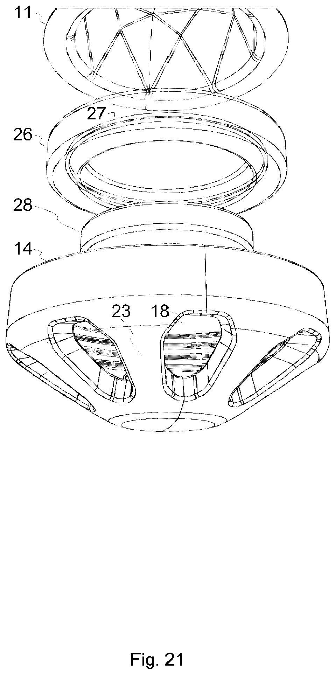

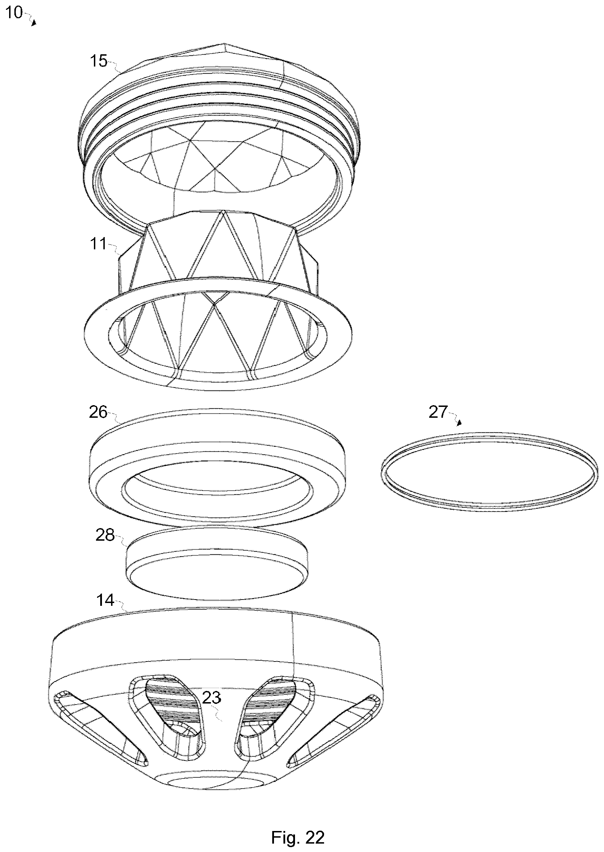

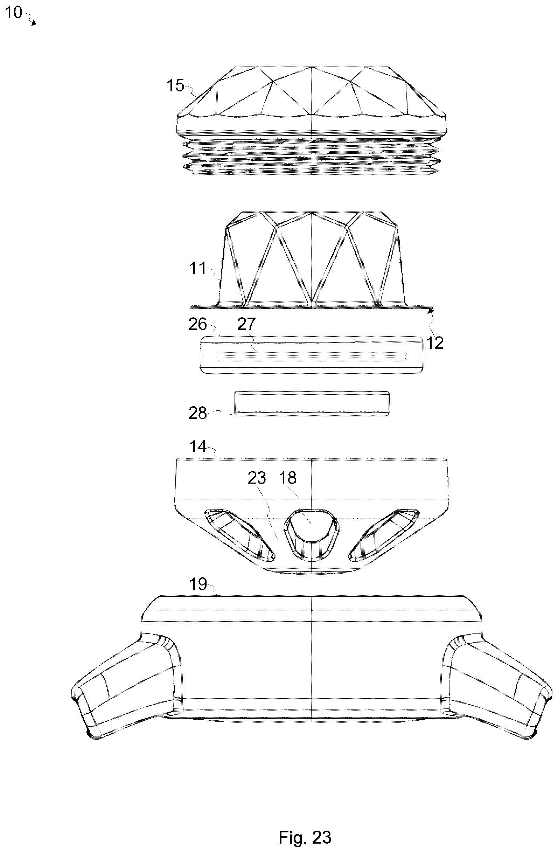

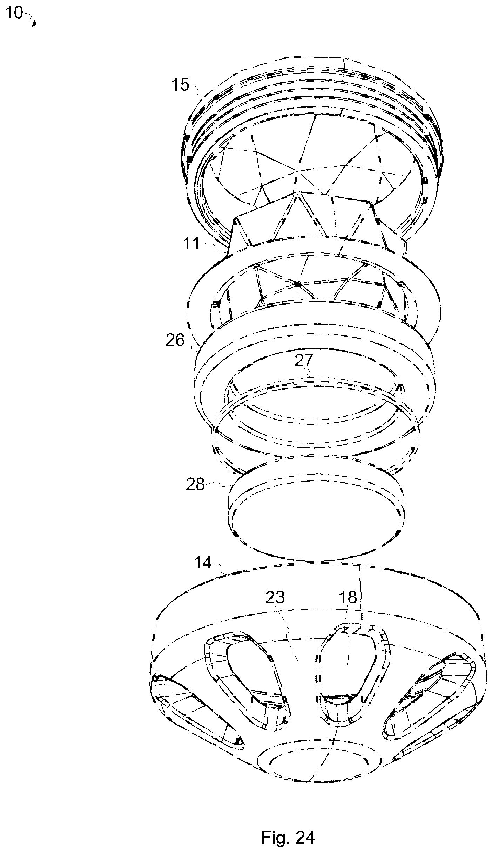

Figure 21 through Figure 24 depict a capsular device (10) that does not depend on the wearer's body heat for promoting evaporation. To this end, a heating module (26) containing a resistor wire (27) powered by a round cell (28) - such as of type CR1220 - is arranged within the retainer (14) beneath the blister pack (11) and adjacent to the latter's membrane (12). A switch (not depicted) may be provided to activate the module (26) only on demand.

### Industrial applicability

The invention is applicable, among others, throughout the health and cosmetic industries.

### Reference signs list

- 10: Device
- 11: Container
- 12: Membrane
- 13: Seal
- 14: Retainer
- 15: Capsule
- 16: Bezel
- 17: Stone
- 18: Gap
- 19: Receptacle
- 20: Bracelet
- 21: Opening
- 22: Duct
- 23: Stay
- 24: Wristband
- 25: Clasp
- 26: Module
- 27: Wire
- 28: Cell

### Citation list

The following documents are cited hereinbefore.

### Patent lite rature

PTL1: EP 19193059 A (LAHME, URSULA) 22.08.2019

### Non-patent literature

NPL1: OBERMAYER, AS, et al. Controlled release technologies: methods, theory, and applications. Edited by KYDONIEUS, AF. Boca Raton, FL: CRC Press, 1980. ISBN 0849356415. p.235-259.

## Claims

1. Preferably capsular device (10) having
a container (11), such as a blister pack (11), containing a preferably gelatinous fragrance and
a preferably microporous membrane (12) covering the container (11) for evaporating the fragrance.

2. Device (10) as per Claim 1, further having
a removable, preferably peelable, seal (13) covering the membrane (12) for sealing the container (11) before use.

3. Device (10) as per Claim 1 or Claim 2, further having
a preferably plastic retainer (14) for retaining the container (11), wherein the membrane (12) faces the retainer (14).

4. Device (10) as per Claim 3, further having
a transparent or opaque capsule (15), such as of glass or polycarbonate, encapsulating the container (11) opposite the membrane (12) and joined to, for example, screwed into, the retainer (14).

5. Device (10) as per Claim 3, further having
a bezel (16) joined, for example, screwed or clipped via the container (11), to the retainer (14) opposite the membrane (12) and encompassing a gemstone or rhinestone (17) for adorning the device (10).

6. Device (10) as per Claim 3, further having
an elastic ring snapped onto or into the retainer (14) opposite the membrane (12) and
a gemstone or rhinestone (17) having a circumferential groove for snapping into the ring.

7. Device (10) as per any of Claim 3 through Claim 6, further having
a heating module (26), preferably containing a resistor wire (27), arranged adjacent to the membrane (12) within the retainer (14) and
a preferably round cell (28) for powering the module (26).

8. Device (10) as per any of Claim 3 through Claim 7, further having gaps (18) formed in the retainer (14) for releasing the evaporated fragrance from the device (10).

9. Jewelry having
a device (10) as per Claim 8 and
a preferably metallic receptacle (19) for removably receiving the device (10).

10. Jewelry as per Claim 9 wherein
the device (10) snap-fits the receptacle (19) and
a preferably central opening (21) is formed in the receptacle (19) for removing the device (10) therefrom.

11. Jewelry as per Claim 9 or Claim 10, further having
air ducts (22) formed in the receptacle (19) for discharging the released fragrance, the ducts (22) preferably extending alongside the device (10) and opening out peripherally opposite the membrane (12).

12. Jewelry as per Claim 11 wherein
the receptacle (19) supports the device (10) pivotably, preferably about a common longitudinal axis of the retainer (14) and receptacle (19).

13. Jewelry as per Claim 12 wherein
the retainer (14) form-fits the receptacle (19) and exhibits stays (23) betwixt the gaps (18), the gaps (18) and stays (23) being arranged vis-à-vis the ducts (22) such that,
in a fragrant pivotal position of the device (10) within the jewelry, the gaps (18) align with the ducts (22) to release the fragrance whereas,
in a nonfragrant pivotal position of the device (10) within the jewelry, the gaps (18) misalign with and the stays (23) block the ducts (22) to withhold the fragrance.

14. Jewelry as per any of Claim 9 through Claim 13 wherein
the retainer (14) bulges outward away from the membrane (12) and
the receptacle (19) bulges inward complementarily to the retainer (14).

15. Bracelet (20) as per any of Claim 9 through Claim 14, having
a preferably resizable wristband (24) attached bilaterally to the receptacle (19) and
a preferably magnetic clasp (25) fastened to the wristband (24).
